# FASCICULE DE BREVET EUROPEEN

(11) **EP 1 140 810 B1**
(45) Date de publication et mention de la délivrance du brevet: **17.03.2004**
(21) Numéro de dépôt: 00902587.5
(22) Date de dépôt: 12.01.2000
(51) Int. Cl.: C07C 311/58, C07C 311/51, C07C 311/59, C07C 311/60, C07C 311/64, C07C 317/36, C07C 335/42, C07D 295/08, C07D 295/21, C07D 307/66, A61K 31/18, A61K 31/64

(54) **DERIVES DE SULFONAMIDES BENZENIQUES ET LEURS UTILISATIONS**
BENZOLSULFONAMID-DERIVATE UND IHRE VERWENDUNG
BENZENIC SULPHONAMIDE DERIVATIVES AND THEIR USES

(30) Priorité: 15.01.1999 BE 9900026
(43) Date de publication de la demande: 10.10.2001
(73) Titulaire: Université de Liège, 4000 Liège (BE)
(72) Inventeur: DELARGE, Jacques, B-4140 Sprimont (BE); DOGNE, Jean-Michel, B-4030 Grivegnée (BE); MASEREEL, Bernard, B-4530 Fize-Fontaine (BE)
(86) Numéro de dépôt international: PCT/EP2000/000225
(87) Numéro de publication internationale: WO 2000/042004

(56) Documents cités:
- EP-A- 0 044 807
- EP-A- 0 365 183
- DE-A- 4 041 780
- P. WANGEMANN, ET AL.: "Chloride-channel blockers in the thick ascending limb of the loop of Henle. Structure activity relationship" PFLUEGERS ARCHIV - EUROPEAN JOURNAL OF PHYSIOLOGY, vol. 407, Suppl. 2, 1986, pages S128-S141, XP002114292 Springer Verlag, Berlin., DE ISSN: 0031-6768
- M. WITTNER, ET AL.: "Analogues of torasemide - structure function realtionships - experiments in the thick ascending limb of the loop of Henle of rabbit nephron" PFLUEGERS ARCHIV - EUROPEAN JOURNAL OF PHYSIOLOGY, vol. 408, no. 1, janvier 1987 (1987-01), pages 54-62, XP002114293 Springer Verlag, Berlin., DE ISSN: 0031-6768

## Description

### Domaine technique

La présente invention est relative à de nouveaux dérivés de sulfonamides benzéniques et à leurs sels non toxiques ainsi qu'à leurs utilisations thérapeutiques.

### Exposé de l'invention

Les nouveaux dérivés de sulfonamides benzéniques, suivant l'invention, sont représentés par la formule générale (I) : 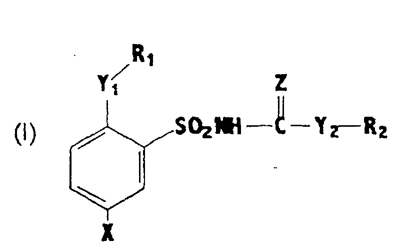 dans laquelle :
X représente un groupe nitro, cyano, halogéno, éventuellement radioactif.
Y₁ représente un groupe aminé secondaire ou tertiaire, du soufre ou de l'oxygène;
Y₂ représente un groupe -NH, ou de l'azote;
Z représente de l'oxygène, du soufre, -N-CN ou -CH-NO₂;
et R₁ et R₂, qui peuvent être identiques ou différents, représentent chacun indépendamment un groupe alkyle linéaire ou ramifié, saturé ou insaturé, de 2 à 12 atomes de carbone, un groupe alicyclique, saturé ou insaturé, de 3 à 12 atomes de carbone, éventuellement radioactif, un groupe aryle, substitué ou non par un ou plusieurs groupes alkyle en C₁-C₄, nitro, cyano, trifluorométhyle, carboxy et halogène, ou un groupe arylalkyle,
ou bien R₁ et/ou R₂ forment avec Y₁ et/ou Y₂ un groupe hétérocyclique de 5 à 7 chaînons, saturé ou insaturé.
à l'exception des dérivés pour lesquels X est un groupe nitro, Y₁ représente un groupe amine secondaire (-NH-), Y₂ représente un groupe -NH Z un oxygène, R₂ un isopropyle et R₁ un élément sélectionné dans le groupe constitué de (*m*-toluyle, phenyle et cyclooctyle) et à l'exception du N-[(2-CYCLOOCTYLAMINO-5-CYANOBENZENE)SULFONYL] N'-ISOPROPYL UREE.

La présente invention se rapporte égaiement aux isomères optiques des dérivés de sulfonamides benzéniques couverts par la formule (I) ou aux sels pharmaceutiquement acceptable de ces dérivés

La présente invention se rapporte également aux sels de ces dérivés, couverts par la formule (I), par addition de bases non toxiques, par exemple aux sels sodiques et potassiques, aux sels avec un acide organique, comme un acide aminé tel que la lysine, l'arginine, par exemple.

Lorsque, dans la formule générale (I), on a un atome de carbone asymétrique (tel que par exemple dans le cas où R₁ et/ou R₂ représentent un groupe arylalkyle), l'invention se rapporte aussi bien aux isomères optiques purs qu'au mélange racémique.

Des classes préférées de composés suivant la formule (I) sont, notamment, celles dans laquelle le X représente un groupe nitro, cyano, bromo, iodo. Y₁ représente un groupe -NH, Y₂ représente un groupe -NH ou un atome d'oxygène et R₁ et R₂ représentent chacun indépendamment un groupe éthyle, butyle, tert-butyle, propyle, isopropyle, pentyle, hexyle, heptyle, octyle, décyle, amyle, cyclopropyle, cyclobutyle, cyclopentyle, cyclohexyle, cycloheptyle, cyclooctyle, cyclododécyle, 2-cyclohexènyle, m-toluyle, o-toluyle, p-toluyle, phényle, allyle, adamantyle, norbornyle, caproyle, 3-carboxyphényle, 2,3-diméthylphényle, 2,4-diméthylphényle, 2,5-diméthylphényle, 2,6-diméthylphényle, 3,4-diméthylphényle, 3,5-diméthylphényle, 2,4,6-triméthylphényle, furfuryle, benzyle ou 1-phényléthyle.

Une autre classe préférée de ces composés est celle dans laquelle R₂ et Y₂ forment un groupe homopipéridino et celle dans laquelle R₁ et Y₁ forment un groupe morpholino ou homopipéridino.

Encore une autre classe particulièrement intéressante est celle constituée par les dérivés radioactifs de l'invention, et notamment les dérivés dans lesquels X représente de l'iode radioactif, tel que le ¹²⁶I et ses isotopes radioactifs ¹²⁵I et ¹³¹I, et ceux dans lesquels R₁ représente un groupe alicyclique saturé ou insaturé avec un hydrogène tritié en positions 2 et /ou 3 du cycle.

Comme on le verra ci-après d'une manière plus détaillée, les dérivés répondant à la formule (I) s'avèrent très utiles dans la prévention et/ou le traitement des maladies impliquant le thromboxane A₂ à différents niveaux, et notamment dans les domaines cardio-vasculaires et sanguins, pulmonaires, de la reproduction et rénaux. Ils constituent également un excellent outil pharmacologique radiomarqué des récepteurs au thromboxane A₂.

La présente invention concerne, par conséquent. également l'utilisation de ces dérivés de sulfonamides benzéniques et leurs sels pour la fabrication de médicaments pour le traitement et/ou la prévention des maladies impliquant le thromboxane A₂ ainsi que comme outils pharmacologiques radiomarqués des récepteurs au thromboxane A₂ et des compositions pharmaceutiques contenant ces dérivés, ces derniers ou leurs sels étant utilisés seuls ou en combinaison avec des excipients et/ou d'autres agents thérapeutiques ayant une activité similaire ou différente.

Les composés actifs de l'invention peuvent être administrés, suivant l'invention, sous forme d'une composition pharmaceutique, en association avec différents excipients pharmaceutiques et cela par voie orale, parentérale, rectale et topique.

Pour l'administration orale, on utilisera des dragées, granulés, tablettes, capsules, solutions, sirops, émulsions et suspensions contenant des excipients ou additifs classiques en pharmacie clinique.

Par voie parentérale, les sels des produits actifs pourraient être administrés en solution aqueuse par exemple.

Pour l'administration par voie rectale, on utilisera des suppositoires et, par voie topique, des lotions, onguents, pommades, aérosols ou nébuliseurs.

Les produits actifs peuvent être utilisés seuls ou en combinaison avec d'autres produits actifs ayant une activité similaire ou différente.

Parmi les composés qui donnent. au point de vue pharmaceutique, des résultats particulièrement intéressants, il y a lieu de retenir ceux dans la formule (I), dans laquelle X représente un groupe NO₂ ou iodo,
Y₁ représente un groupe aminé secondaire,
Y₂ représente un groupe -NH,
Z représente un groupe oxygène, soufre ou -N-CN,
et R₁ représente un groupe cyclohexyle, cycloheptyle ou cychlohexèn-2-yle, et
R₂ un groupe isopropyle, tert-butyle, pentyle ou homopipéridino,
et de citer tout particulièrement les composés :
N-[(2-cyclohexylamino-5-nitrobenzène)sulfonyl]N'-tert-butyl urée,
N-cyano-N'-[(2-métatoluylamino-5-nitrobenzène)sulfonyl]homopipéridinoamidine,
N-[(2-cycloheptylamino-5-nitrobenzène)sulfonyl]N'-cyclohexyl thiourée, et
N-[(cyclohexèn-2-yl)-5-iodobenzène)sulfonyl]N'-pentyl urée.

### Meilleure manière de réaliser l'invention

On donne ci-après les définitions et explications relatives à la synthèse des dérivés de l'invention.

L'évolution de la plupart des réactions est suivie par chromatographie sur couche mince (C.C.M.). Les plaques sont constituées de feuilles d'aluminium recouvertes de gel de silice 60F₂₅₄ (Merck®). La plaque est examinée aux rayons ultraviolets à 254 ou 362 nm.

Les analyses élémentaires (C, H, N, S) ont été réalisées et correspondent à la formule théorique (+/-0,4%). Les spectres IR et [¹H]-RMN sont en accord avec les formules proposées.

Les analyses élémentaires (C, H, N, S) ont été déterminées sur un analyseur Carlo Erba EA 1108.

Les spectres infrarouges des différentes substances (1 mg) ont été enregistrés à l'aide d'un FT-IR Perkin-Elmer 1750 sous forme de pastilles de KBr (250 mg).

Après dissolution dans le DMSO deutérié, le spectre RMN-¹H des différentes molécules est enregistré sur un appareil Bruker 400.

Les points de fusion des molécules obtenues ont été déterminés sur un appareil Büchi-Tottoli.

Les composés de formule générale (I) peuvent être obtenus aisément de plusieurs manières différentes résumées dans les schémas de synthèse ci-après.

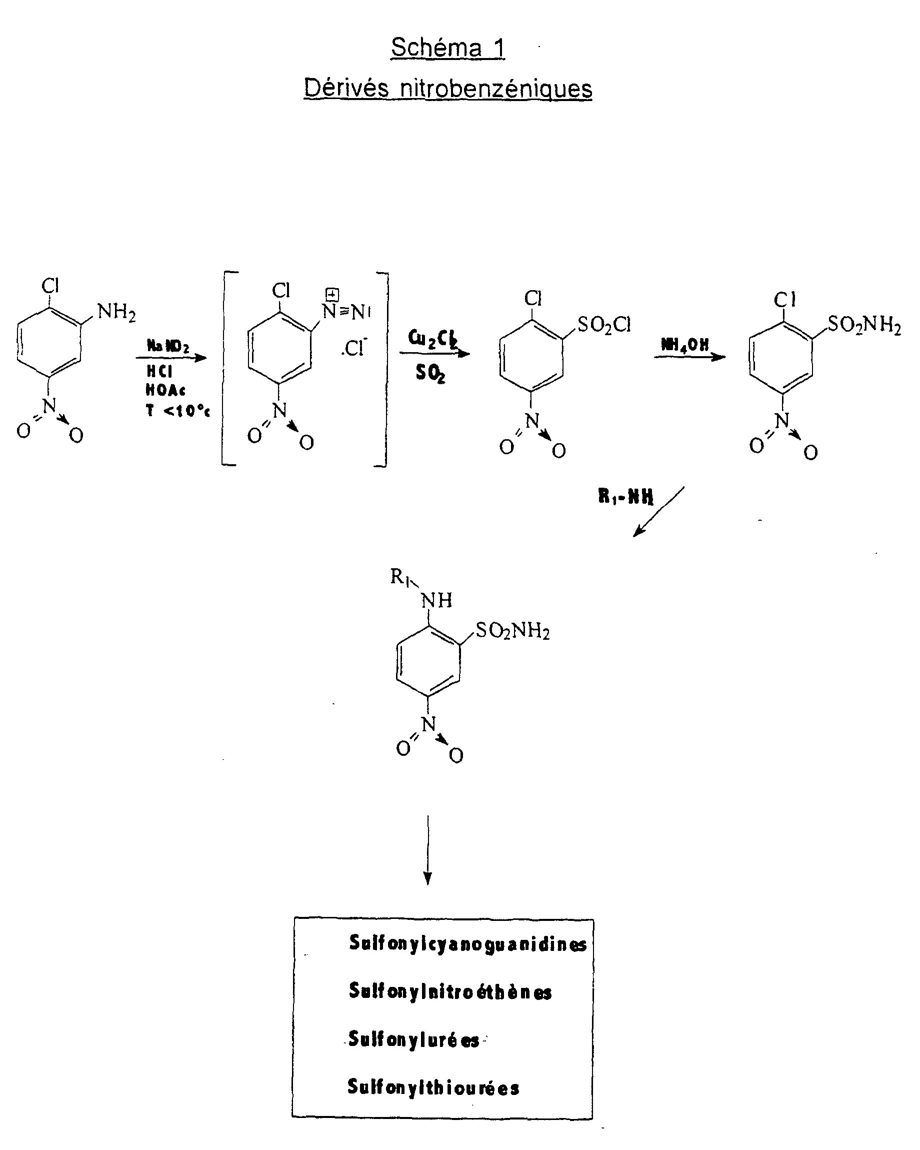

La 2-chloro-5-nitroaniline est diazotée à une température comprise entre 0 et 10°C. Le sel de diazonium formé est substitué en présence de sels cuivreux (catalyseur) par de l'anhydride sulfureux pour générer le sulfochlorure qui en présence d'ammoniaque forme le 2-chloro-5-nitrobenzènesulfonamide correspondant. Le chlore est ensuite substitué par une amine adéquate.

Les fonctions sulfonylurées, thiourées, cyanoguanidines et nitroéthènes adéquates s'obtiennent par condensation de réactifs choisis (isocyanates pour les sulfonylurées ou isothiocyanates pour les sulfonylthiourées) ou préparés [N-cyano-N'-alkyl (ou aryl)carbamimidothioate de S-méthyle pour les sulfonylcyanoguanidines et 1-alkyl (ou aryl)amino-1'-méthylthio-2-nitroéthylène pour les sulfonitroéthènes] sur le sel sodique de sulfonamide obtenu par réaction avec exactement 1 équivalent de soude.

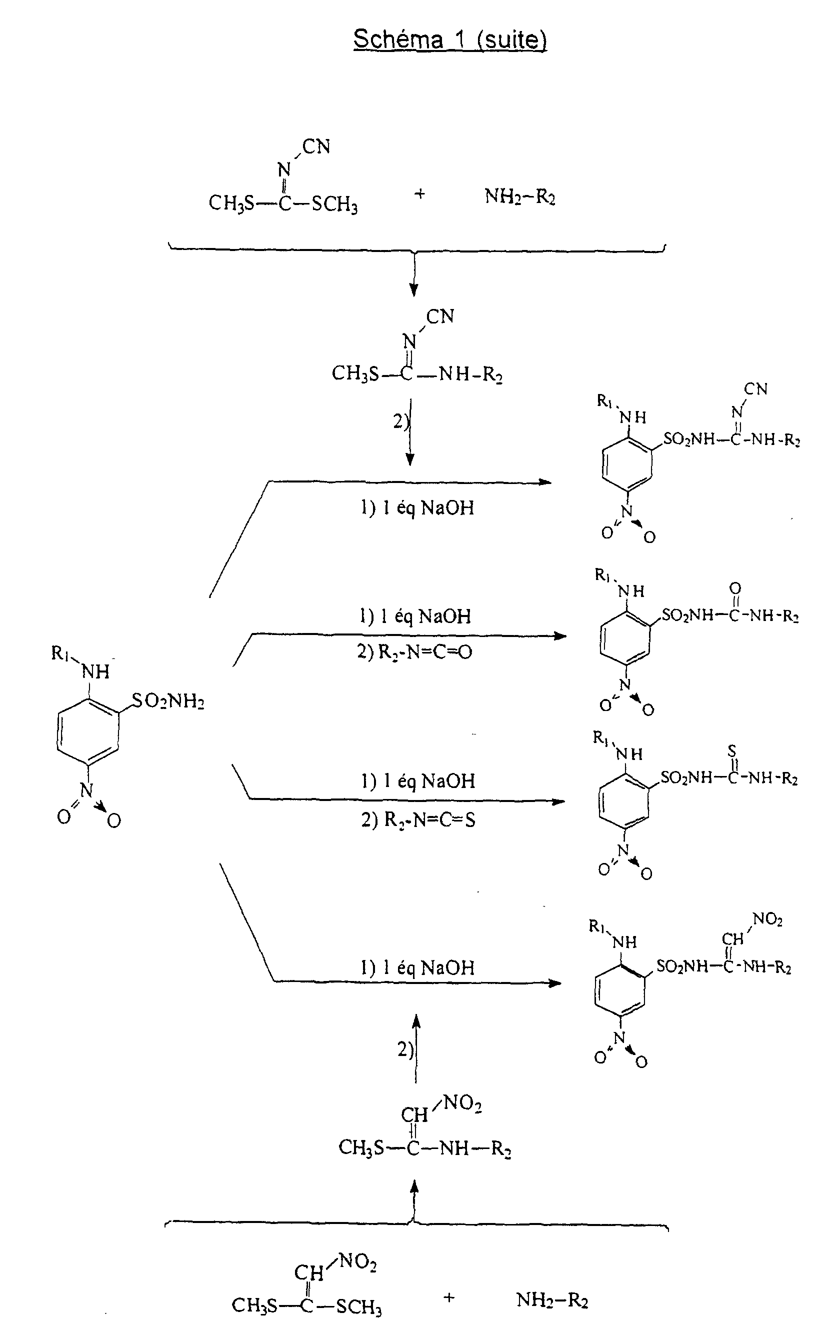

### 1.1.) 2-Chloro-5-nitrobenzènesulfonamide

D'une part, on sature 160 ml d'acide acétique glacial en SO₂ durant 5 heures (solution A), d'autre part, 10 g de 2-chloro-5-nitroaniline sont dissous dans 40 ml d'acide chlorhydrique 12 N et 100 ml d'acide acétique glacial (solution B). Cette solution est refroidie jusqu'à atteindre une température voisine de 0 à -5°C. Enfin, on dissout 7 g de nitrite sodique dans 10 ml d'eau (solution C). La solution C est ajoutée goutte à goutte à la solution B pour former le sel de diazonium. La température doit être maintenue vers -5°C. 4 g de CuCl₂ sont dissous dans 10 ml d'eau (solution D). La solution D est ajoutée à la solution A et agitée 2 minutes. Un précipité de Cu₂Cl₂ apparaît. La solution de diazonium est ensuite ajoutée prudemment et sous agitation à cette suspension puis 180 g de glace sont additionnés au milieu réactionnel. Le précipité de chlorure de sulfonyle est recueilli rapidement sur filtre, lavé à l'eau glacée et additionné sous agitation à une solution préalablement refroidie, constituée de 50 ml d'ammoniaque concentré et de 100 ml d'eau. Après filtration, le filtrat est clarifié au charbon, puis concentré sous pression réduite. Le pH est ajusté à 5-6 par de l'acide chlorhydrique 10 N. Après refroidissement, le sulfonamide est recueilli sur filtre, lavé à l'eau et séché. Il est éventuellement recristallisé dans le méthanol.
- Rendement :: 50-60%.
- Point de fusion :: 178°C
- Poids moléculaire :: 236,62 (C₆H₅ClN₂O₄S)

### 1.2.) 2-Alkyl (ou aryl)amino-5-nitrobenzènesulfonamides

10 g de 2-chloro-5-nitrobenzènesulfonamide préparé en 1.1.) sont mis en solution dans 50 ml de 3-chlorotoluène avec 15 ml d'amine. On chauffe environ 3 heures à reflux, sous azote. La réaction est suivie par chromatographie en couche mince. Au terme, la solution est filtrée à chaud, puis concentrée sous pression réduite. Le résidu est repris par une solution de soude à 2% et purifiée au charbon. On amène à pH 1 par de l'acide chlorhydrique 2N. La suspension est extraite trois fois par 100 ml d'éther diéthylique. L'éther est évaporé sous pression réduite. Le résidu est repris par une solution de soude à 2%, puis clarifié au charbon et amené à pH 7,5-8 par de l'acide chlorhydrique 5N.

Le précipité de 2-alkyl (ou aryl)amino-5-nitrobenzènesulfonamide est recueilli sur filtre, lavé et recristallisé dans le méthanol.

### 1.3.) Sulfonylurées

### N-[(2-alkyl (ou aryl)amino-5-nitrobenzène)sulfonyl] N'-alkyl (ou aryl) urées

On dissout 0,01 mole de sulfonamide approprié préparé en 1.2.) dans 30 ml d'un mélange eau-acétone (50/50 vol/vol). Après avoir additionné un équivalent de soude (solution à 10%), on ajoute 0,02 mole d'isocyanate adéquat. Pour les isocyanates peu volatils (P.E. >90°C), la solution est portée à reflux sous agitation alors que pour les isocyanates volatils (isopropyl-, éthyl-, méthylisocyanate), la solution est mise sous agitation à température ambiante. La progression de la réaction est suivie par chromatographie sur couche mince. Au terme, le milieu réactionnel est évaporé sous dépression, le résidu est repris par 100 ml de soude à 2%. Cette solution est extraite trois fois par 150 ml d'éther diéthylique puis clarifiée au charbon. La phase aqueuse est amenée à pH 7,5 par de l'acide chlorhydrique 2N. La sulfonylurée qui précipite est recueillie sur filtre, lavée à l'eau et séchée. Le produit est éventuellement recristallisé dans l'alcool dilué.
Exemples de composés préparés suivant ce procédé (Tableau 1) :
n° s 1; 2; 13; 17; 19; 20; 21; 22; 23; 25; 26; 27; 28; 29; 30: 31; 32; 33; 34; 44; 46; 47; 48; 49; 50; 52; 53; 54; 55; 56; 57; 58; 59; 60; 61; 62; 63; 64; 65; 67; 73; 75; 76; 77; 78; 79; 80; 81; 82; 83; 84; 85; 86; 87; 88: 89; 90; 91; 92; 94; 95; 96.

### 1.4.) Sulfonylthiourées

### N-[(2-alkyl (ou aryl)amino-5-nitrobenzène)sulfonyl] N'-alkyl (ou aryl) thiourées

On dissout 0,01 mole de sulfonamide approprié préparé en 1.2.) dans 30 ml d'un mélange eau-acétone (50/50 vol/vol). Après avoir additionné un équivalent de soude (solution à 10%), on ajoute 0,02 mole d'isothiocyanate adéquat. Pour les isothiocyanates peu volatils (P.E. >90°C), la solution est portée à reflux sous agitation alors que pour les isothiocyanates volatils (isopropyl-, éthyl-, méthylisothiocyanate), la solution est mise sous agitation à température ambiante. La progression de la réaction est suivie par chromatographie sur couche mince. Au terme, le milieu réactionnel est évaporé sous dépression, le résidu est repris par 100 ml de soude à 2%. Cette solution est extraite trois fois par 150 ml d'éther diéthylique puis clarifiée au charbon. La phase aqueuse est amenée à pH 7,5 par de l'acide chlorhydrique 2N. La sulfonylthiourée qui précipite est recueillie sur filtre, lavée à l'eau et séchée. Le produit est éventuellement recristallisé dans l'alcool dilué.
Exemples de composés préparés suivant ce procédé (Tableau 1) :
n° s 11; 12; 14; 15; 16; 35; 36; 37; 38; 39; 40; 41; 50.

### 1.5.) Sulfonylcyanoguanidines

### 1.5.1.) N-cyano-N'-alkyl (ou aryl)carbamimidothioates de S-méthyle

On fait réagir 0,05 mole de N-cyanodithioiminocarbonate de diméthyle avec 0,075 mole d'amine adéquate dans 10 ml d'éthanol. Cette solution est portée à reflux pendant 15 à 20 heures (pour les amines volatiles, la réaction se déroulera à température ambiante). La progression de la réaction est suivie par chromatographie sur couche mince. Au terme, la solution est refroidie sous eau glacée et le précipité recueilli sur filtre, puis recristallisé dans le méthanol bouillant.

### 1.5.2.) N-[(2-alkyl (ou aryl)amino-5-nitrobenzène)sulfonyl] N'-alkyl cyanoguanidines

On dissout 0,01 mole de sulfonamide approprié préparé en 1.2.) dans 5 ml d'un mélange eau-acétone (50/50 vol/vol) puis on ajoute 0,01 mole de soude (solution à 10%). Cette solution est mise sous agitation 10 minutes puis concentrée sous pression réduite. Le résidu (sulfonamidate) est solubilisé dans un mélange constitué de 3 ml de dioxane et 2 ml de diméthylformamide puis additionné de 0,015 mole de N-cyano-N'-alkylcarbamimidothioate de S-méthyle adéquat préparé en 1.5.1.). Cette solution est portée à reflux sous agitation. La progression de la réaction est suivie par chromatographie sur couche mince. Au terme de la réaction, la solution est concentrée sous pression réduite puis additionnée de 100 ml de soude à 2%. Cette solution est extraite trois fois par 150 ml d'éther diéthylique puis clarifiée au charbon. La phase aqueuse est amenée à pH 7,5 par de l'acide chlorhydrique 2N. Le précipité est recueilli sur filtre, lavé à l'eau et séché. Le produit est éventuellement recristallisé dans le méthanol.
- C.C.M. :: acétate d'éthyle 13/cyclohexane 7.
Exemples de composés préparés suivant ce procédé (Tableau 1) :
n° s 3; 4; 5; 6; 7; 8; 9; 18; 51; 74.

### 1.6.) Sulfonylnitroéthènes

### 1.6.1.) 1-Alkyl (ou aryl)amino-1'-méthylthio-2-nitroéthylènes

On fait réagir 0,05 mole de 1,1'-bis(méthylthio)-2-nitroéthylène avec 0,075 mole d'amine adéquate dans 10 ml d'éthanol. Cette solution est portée à reflux pendant 15 à 20 heures (pour les amines volatiles, la réaction se déroulera à température ambiante). La progression de la réaction est suivie par chromatographie sur couche mince. Au terme, la solution est refroidie sous eau glacée et additionnée de 30 ml d'eau. Le précipité obtenu est recueilli sur filtre, puis recristallisé dans le méthanol bouillant.
- C.C.M.:: acétate d'éthyle 8/éther de pétrole PE 40/60 12.

### 1.6.2.) 1-Alkyl (ou aryl)amino-1'-[2-alkyl (ou aryl)amino-5'-nitrobenzènesulfonamido]-2-nitroéthylènes

On dissout 0,01 mole de sulfonamide approprié préparé en 1.2.) dans 5 ml d'un mélange eau-acétone (50/50 vol/vol) puis on ajoute 0,01 mole de soude (solution à 10%). Cette solution est mise sous agitation 10 minutes puis concentrée sous pression réduite. Le résidu (sulfonamidate) est solubilisé dans un mélange constitué de 3 ml de dioxane et 2 ml de diméthylformamide puis additionné de 0,015 mole de 1-alkyl (ou aryl)amino-1'-méthylthio-2-nitroéthylène adéquat préparé en 1.6.1). Cette solution est portée à reflux sous agitation. La progression de la réaction est suivie par chromatographie sur couche mince. Au terme de la réaction, la solution est concentrée sous pression réduite puis additionnée de 100 ml de soude à 2%. Cette solution est extraite trois fois par 150 ml d'éther diéthylique puis clarifiée au charbon. La phase aqueuse est amenée à pH 7,5 par de l'acide chlorhydrique 2N. Le précipité est recueilli sur filtre, lavé à l'eau et séché. Le produit est éventuellement recristallisé dans le méthanol.
- C.C.M. :: acétate d'éthyle 8/éther de pétrole PE 40/60 12.
Exemple de composé préparé suivant ce procédé (Tableau 1) :
n° 10.

### 1.7.) Sulfonylcarbamates

### 2-Alkyl (ou aryl)amino-5-nitrobenzénesulfonylcarbamates d'éthyle

On met en solution 0,01 mole de sulfonamide préparé en 1.2.) dans 10 ml de pyridine anhydre. Sous agitation, on ajoute goutte à goutte un large excès (10 ml) de chloroformiate d'éthyle. L'évolution de la synthèse est suivie par chromatographie sur couche mince. Au terme de la réaction, environ 15 minutes après avoir ajouté le chloroformiate, la solution est évaporée sous pression réduite et le résidu repris par 100 ml de soude à 2%. Après deux extractions par 150 ml d'éther diéthylique, la solution alcaline est clarifiée au charbon puis neutralisée à pH 6,5 par de l'acide chlorhydrique 2N. Le carbamate précipité est recueilli, lavé à l'eau et séché sous vide.
- Rendement :: 75%-88%
- C.C.M. :: acétate d'éthyle, méthanol et triéthylamine 18/2/1.
Exemple de composé préparé suivant ce procédé (Tableau 1) :
n° 45.

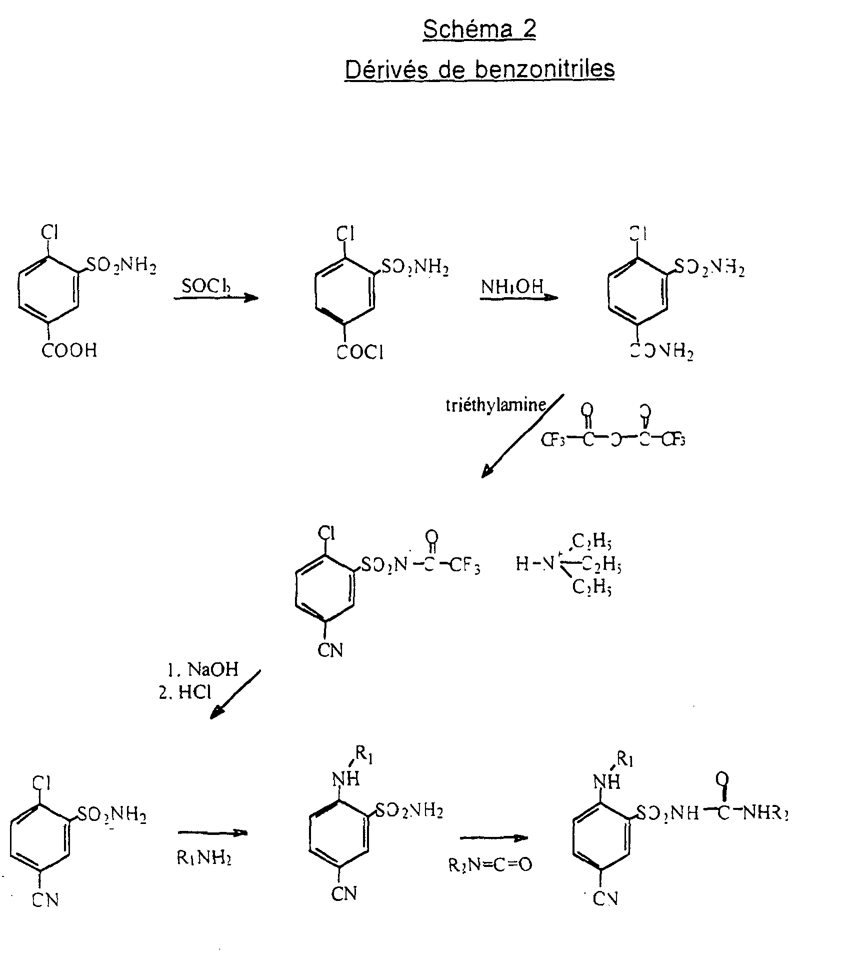

L'acide 4-chloro-3-sulfamoylbenzoïque est mis en réaction avec le chlorure de thionyle pour former le chlorure d'acide qui, en présence d'ammoniaque, génère le carboxamide correspondant. Ce dernier est déshydraté en présence d'anhydride trifluoroacétique. Le sulfonamide acylé lors de cette réaction est hydrolysé en présence d'exactement 2,5 équivalents de soude. Le sulfonamide est ensuite régénéré à pH acide. Le chlore est alors substitué par une amine adéquate. La fonction sulfonylurée s'obtient par condensation de l'isocyanate choisi sur le sel sodique de sulfonamide préalablement préparé par réaction avec exactement 1 équivalent de soude. La fonction carboxylique est ensuite régénérée par hydrolyse alcaline du benzonitrile.

### 2.1.) 4-Chloro-3-sulfamoylbenzènecarboxamide

On fait réagir 0,01 mole d'acide 4-chloro-3-sulfamoylbenzoïque avec 25 ml de chlorure de thionyle. Cette solution est portée à reflux pendant 3 heures. Au terme, le milieu réactionnel est concentré sous pression réduite, puis additionné de 10 ml de dioxane. Cette solution est ajoutée sous agitation à une solution préalablement refroidie constituée de 25 ml d'ammoniaque concentré et de 50 ml d'eau. L'excès d'ammoniaque est éliminé sous pression réduite. Le précipité est recueilli sur filtre, lavé à l'eau et séché. Il est éventuellement recristallisé dans le méthanol.
- Rendement :: 50-60%
- Point de fusion :: 220-222°C
- Poids moléculaire :: 234,656 (C₇H₇ClN₂O₃S).
- C.C.M. :: acétate d'éthyle 18/méthanol 4/acide formique 5 gouttes.

### 2.2.) 4-Chloro-3-sulfamoylbenzonitrile

On ajoute à 0,01 mole de 4-chloro-3-sulfamoylbenzènecarboxamide 80 ml de tétrahydrofuranne anhydre. Cette suspension est refroidie à 0°C puis additionnée successivement de 0,045 mole de triéthylamine et de 0,02 mole d'anhydride trifluoracétique. La progression de la réaction est suivie par chromatographie sur couche mince. Au terme. le milieu réactionnel est concentré sous dépression. Le résidu est repris par de l'eau, filtré et lavé. Le produit obtenu est mis en réaction avec 2.5 équivalents d'une solution de soude 2N pendant maximum 30 minutes. La solution est ensuite amenée à pH 1 par de l'acide chlorhydrique 2N. Le précipité est ensuite recueilli rapidement sur filtre, lavé à l'eau et séché.
- Rendement :: 70-80%
- Point de fusion :: 199-201°C
- Poids moléculaire :: 216.64 (C₇H₅ClN₂O₂S).
- Analyse élémentaire :: trouvée : +/- 0,4% de calculée.
- C.C.M. :: acétate d'éthyle 18/méthanol 4/acide formique 5 gouttes.

### 2.3.) 4-Alkyl (ou aryl)amino-3-sulfamoylbenzonitriles

On procède comme en 1.2.) en utilisant le 4-chloro-3-sulfamoylbenzonitrile comme matière première.

### 2.4.) N-[(2-alkyl (ou aryl)amino-5-cyanobenzène)sulfonyl] N'-alkyl (ou aryl)urées

On procède comme en 1.3.) en utilisant du 4-alkyl (ou aryl)-amino-3-sulfamoylbenzonitrile comme matière première.
Exemples de composés préparés suivant ce procédé (Tableau 1) :
n°s 24; 43; 66: 97.

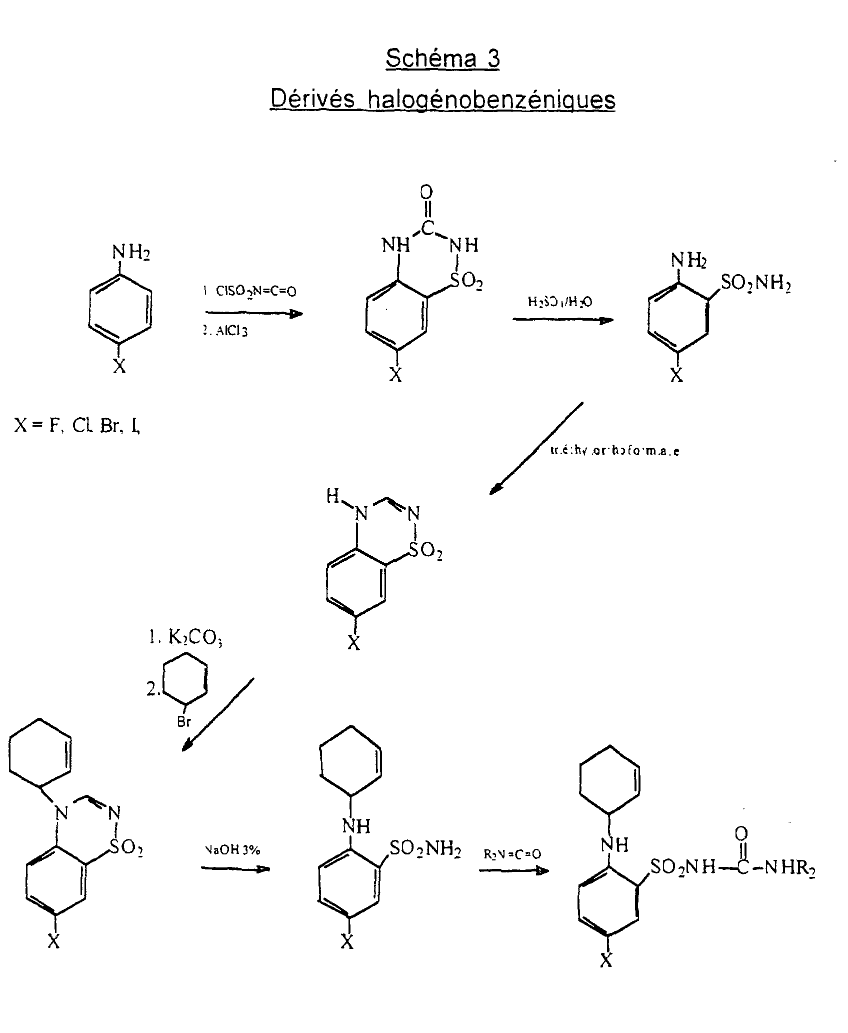

### Dérivés halogénobenzéniques

L'aniline adéquate est mise en réaction avec un léger excès de chlorosulfonylisocyanate à une température de -5°C. Du chlorure d'aluminium est ensuite ajouté au milieu en vue d'obtenir le produit cyclisé suivant : 2,3-dihydro-7-halogéno-3-oxo-4H-1,2,4-benzothiazidine 1,1-dioxyde. Ce dernier est hydrolysé par traitement en milieu sulfurique. L'aminosulfonamide est ensuite engagé dans une nouvelle réaction de cyclisation à l'orthoformiate de triéthyle. Le 7-halogéno-4H-1,2,4-benzothiadiazine 1,1-dioxyde obtenu est alkylé en position 4 par le 3-bromocyclohexène en présence de 4 équivalents de carbonate potassique.
Le 2-(cyclohexène-2-yl)amino-5-halogénobenzènesulfonamide est ensuite généré par traitement à la soude. La fonction sulfonylurée s'obtient par condensation de l'isocyanate choisi sur le sel sodique de sulfonamide préalablement préparé par réaction avec exactement 1 équivalent de soude.

### 3.1.) 2,3-Dihydro-7-halogéno-3-oxo-4H-1,2,4-benzothiadiazine 1,1-dioxydes

On solubilise 0,07 mole de chlorosulfonylisocyanate dans 90 ml de nitrométhane préalablement refroidi à -5°C puis on ajoute goutte à goutte 50 ml d'une solution de nitrométhane contenant 0,06 mole d'amine adéquate. On ajoute goutte à goutte 0,097 mole de chlorure d'aluminium au milieu. La solution est chauffée à reflux 45 minutes puis versée sur glace. Le précipité obtenu est recueilli sur filtre. lavé à l'eau et séché. Le produit est éventuellement purifié par redissolution dans une solution aqueuse de bicarbonate sodique (5% m/vol) et reprécipitation par addition d'acide chlorhydrique 2N.
- Rendement :: 70-75%
- C.C.M. :: acétate d'éthyle 20/acide formique 5 gouttes.

### 3.2.) 2-Amino-5-halogénobenzènesulfonamides

On ajoute 0,01 mole de 2,3-dihydro-7-halogéno-3-oxo-4H-1,2,4-benzothiadiazine 1,1-dioxyde préparé en 5.1.) à 100 ml d'un mélange acide sulfurique-eau (50/50). Le milieu réactionnel est porté à reflux une heure. Après refroidissement, la solution est amenée à pH 3 par de la soude à 30%. Le précipité obtenu est recueilli sur filtre, lavé à l'eau et séché.
- Rendement :: 80-85%
- C.C.M. :: acétate d'éthyle 13/cyclohexane 7/acide formique 5 gouttes.

### 3.3.) 7-Halogéno-4H-1,2,4-benzothiadiazine 1,1-dioxydes

On solubilise 0,01 mole de 2-amino-5-halogénobenzènesulfonamide préparé en 5.2.) dans 25 ml d'orthoformiate de triéthyle. Le milieu réactionnel est porté à reflux une heure. Après refroidissement, le précipité est recueilli sur filtre, lavé et séché.
- Rendement :: 50-60%
- C.C.M. :: acétate d'éthyle 13/cyclohexane 7/acide formique 5 gouttes.

### 3.4.) 4-(Cyclohexèn-2-yl)-7-halogéno-1,2,4-benzothiadiazine 1,1-dioxydes

On met en suspension 0,01 mole de 7-halogéno-4H-1,2,4-benzothiadiazine 1,1-dioxyde préparé en 5.3.) dans 300 ml d'acétonitrile contenant 0,04 mole de carbonate potassique. Le milieu réactionnel est porté à reflux 30 minutes puis additionné de 0,04 mole de 3-bromocyclohexène. Le reflux est maintenu durant 4 heures. La réaction est suivie par chromatographie sur couche mince. Au terme, le carbonate potassique en excès est recueilli sur filtre. Le filtrat est concentré sous pression réduite. Le résidu est additionné de 50 ml de méthanol porté à ébullition. Le précipité est recueilli sur filtre, lavé et séché.
- Rendement :: 60-70%
- C.C.M. :: acétate d'éthyle 13/cyclohexane 7/acide formique 5 gouttes.

### 3.5.) 2-(Cyclohexèn-2-yl)amino-5-halogénobenzènesulfonamides

On ajoute à 0,01 mole de 4-(cyclohexèn-2-yl)-7-halogéno-1,2,4-benzothiadiazine 1,1-dioxyde préparé en 5.4.) 50 ml de soude à 3%. La suspension est portée à 60°C pendant douze heures. Au terme, la solution est amenée à pH 7 par de l'acide chlorhydrique 5 N. Le précipité obtenu est recueilli sur filtre, lavé à l'eau et séché.
- Rendement :: 50-60%
- C.C.M. :: acétate d'éthyle 13/cyclohexane 7/acide formique 5 gouttes.

### 3.6.) N-[(2-cyclohexèn-2-yl)-5-halogénobenzène)sulfonyl] N'-alkyl (ou aryl)urées

On procède comme en 1.3.) en utilisant du 2-(cyclohexèn-2-yl)amino-5-halogénobenzènesulfonamide comme matière première.
Exemples de composés préparés suivant ce procédé (Tableau 1) :
n°s 68; 69; 70; 71; 72.

Le Tableau 1 donné ci-après se rapporte à la préparation d'une série de composés répondant à la formule générale (I).

Comme on l'a déjà précisé, les nouveaux dérivés de sulfonamides benzéniques ainsi décrits sont intéressants dans la prévention et/ou le traitement des maladies impliquant le thromboxane A₂ à différents niveaux et notamment :
Cardio-vasculaires et sanguins:
   - Infarctus du myocarde,
   - Formation de thrombus et lésions vasculaires,
   - Troubles de l'hémostase,
   - Athérosclérose,
   - Artériosclérose.
   - Ischémie myocardique,
   - Hypertension artérielle,
Pulmonaires :
   - Asthme,
   - Bronchospasme,
   - Hypertension pulmonaire.
De la reproduction :
   - Prééclampsie.
Rénaux :
   - Hypertension rénale,
   - Dysfonctionnement de la fonction rénale.

Les dérivés de l'invention sont également intéressants en vue de la conception d'un outil pharmacologique radiomarqué original des récepteurs au thromboxane A₂. Le schéma 6 suivant montre ce type d'application au départ des composés n°s 80 et 104 (Tableau 1).

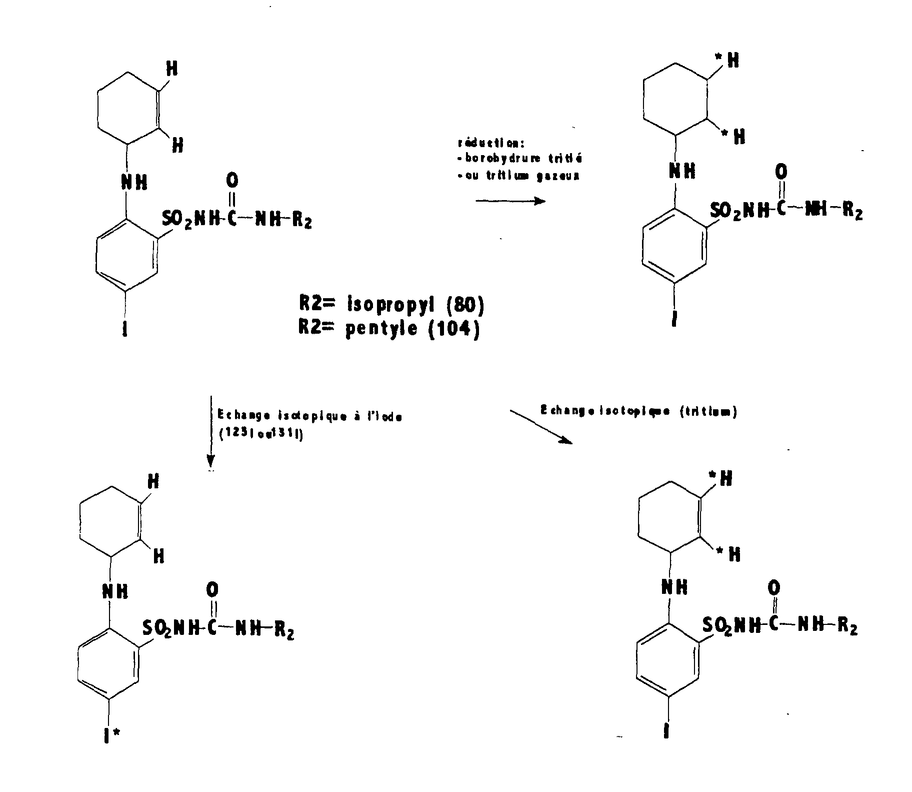

Comme on peut le voir, deux techniques de marquage sont envisagées :
- Une technique de marquage au tritium (³H).
   - soit par réduction avec un réducteur tritié : (hydrogène tritié ou borohydrure tritié).
   - soit par échange isotopique.
- Une technique de marquage à l'iode (¹²⁵I ou ¹³¹I) par échange isotopique.

Ce qui suit et les tableaux ci-après concernent des résultats de tests pharmacologiques réalisés- sur un certain nombre de composés donnés dans le Tableau 1.

Pour opérer une première sélection, on a examiné la capacité de ces composés à déplacer de manière spécifique un ligand tritié, le [³H] SQ-29.548, du récepteur au thomboxane A₂ des plaquettes humaines. Ce test de binding est, en effet. simple, rapide et permet ainsi une sélection du ou des produits qui possèdent une affinité marquée pour les récepteurs plaquettaires (TPα) au thromboxane A₂.

Le pouvoir antagoniste du TXA₂ des composés sélectionnés a été évalué par un test de mesure d'agrégation plaquettaire induite par le U-46619 (agoniste stable du thromboxane A₂) ou l'acide arachidonique.

Deux tests sur musculature lisse ont permis de confirmer le pouvoir antagoniste sur les récepteurs TPτ du thromboxane A₂. En effet, on a estimé la capacité des composés sélectionnés lors du binding à prévenir la contraction du fundus de rat induite par l'U-46619 et à relaxer l'aorte de rat précontractée par ce même agoniste stable du TXA₂.

Tous les résultats sont exposés en parallèle avec ceux de deux antagonistes du thromboxane A₂ décrits dans la littérature et qui ont fait l'objet d'études cliniques approfondies : le sulotroban et la SQ-29.548.

Le SQ-29.548 et le U-46619 sont respectivement l'acide [15-[1-alpha,2-bêta (5Z),3-bêta,4-alpha]-7-[3-[[2-[phénylamino)-carbonyl]hydrazino]méthyl]-7-oxabicyclo[2.2.1]hept-2-yl]-5-hepténoïque et la 9,11-didésoxy,11-alpha,9-alpha-époxy-méthanoprostaglandine F₂ₐ.

Les matériels et méthodes utilisés pour les tests pharmacologiques sont ceux décrits dans la littérature.

**TABLEAU 2**

| Liaison au récepteur du thromboxane A₂ des plaquettes humaines Résultats du test de binding sur plaquettes humaines | | | |
|---|---|---|---|
| Composé numéro | Testde binding | | |
| | 10⁻⁶M : (%)¹affinité | 10⁻⁷M : (%)¹affinité | IC 50² (ηM) |
| Sulotroban | 55,6 | 16,5 | 1100 |
| SQ-29.548 | 100 | 72,0 | 23,2 |
| 1 | 93.6 | 68,0 | |
| 2 | 67,7 | | |
| 3 | 20,1 | | |
| 4 | 50,0 | | |
| 5 | 72,1 | | |
| 6 | 29,8 | | |
| 7 | 42,9 | | |
| 8 | 33,0 | | |
| 9 | 15,4 | | |
| 10 | 57,7 | | |
| 11 | 63,7 | | |
| 12 | 67,2 | | |
| 13 | 97,7 | 60,3 | |
| 14 | 92,9 | 34,0 | |
| 15 | 81,0 | 16.6 | |
| 16 | 100 | 46,1 | |
| 17 | 100 | 88,0 | 22,7 |
| 18 | 100 | 88,9 | 24,2 |
| 19 | 97,8 | 93,3 | 3,96 |
| 20 | 1,6 | | |
| 21 | 92,2 | 44,2 | |
| 22 | 100 | 84,1 | 41,7 |
| 23 | 95,5 | 62,9 | |
| 24 | 73,7 | | |
| 25 | 100 | 95,2 | 10,5 |
| 26 | 94,3 | 93,3 | 16,9 |
| 27 | 79,6 | | |
| 28 | 81,9 | 39,6 | |
| 29 | 97,4 | 95,4 | 7,8 |
| 30 | 95,1 | 80,8 | |
| 31 | 80,5 | 42,2 | |
| 32 | 86,7 | 46,0 | |
| 33 | 86,6 | 52,4 | |
| 34 | 77,3 | | |
| 35 | 45,0 | | |
| 36 | 75,6 | | |
| 37 | 72,3 | | |
| 38 | 77,2 | | |
| 39 | 74,5 | | |
| 40 | 94,4 | 63,0 | 26,9 |
| 41 | 75,9 | | |
| 42 | 92,3 | 50,5 | |
| 43 | 50,0 | | |
| 44 | 80,2 | 51,3 | |
| 45 | 79.9 | 50.4 | |
| 46 | 1,4 | | |
| 47 | 98,7 | 89,4 | |
| 48 | 51,9 | | |
| 49 | 98,3 | 94,9 | 2,0 |
| 50 | 95,7 | 76,0 | |
| 51 | 64,7 | | |
| 52 | 99,0 | 93,9 | 2,8 |
| 53 | 36,5 | | |
| 54 | 91,7 | | |
| 55 | 98,2 | 93,3 | 3,4 |
| 56 | 0,0 | | |
| 57 | 67,0 | | |
| 58 | 83,2 | | |
| 59 | 92,2 | | |
| 60 | 79,1 | | |
| 61 | 98,6 | 94,8 | 1,1 |
| 62 | 3,7 | | |
| 63 | 7,5 | | |
| 64 | 57,8 | | |
| 65 | 46,6 | | |
| 66 | 49,6 | | |
| 67 | 98,3 | 95,8 | 1,3 |
| 68 | 93,2 | 67,4 | |
| 69 | 13,2 | | |
| 70 | 63,8 | | |
| 71 | 77,8 | | |
| 72 | 86,5 | 52,7 | |
| 73 | 98,3 | 95,6 | 1,2 |
| 74 | 90,9 | | |
| 75 | 93,1 | | |
| 76 | 97,6 | 93,5 | 3,5 |
| 77 | 79,4 | | |
| 78 | 95,3 | 71,6 | 4,2 |
| 79 | 96,6 | | |
| 80 | 98,6 | 97,9 | 2,4 |
| 81 | 93,3 | 65,0 | 57,8 |
| 82 | 98,5 | 98,0 | 4,5 |
| 83 | 98,5 | 92,7 | 4,5 |
| 84 | 96,9 | 73.7 | 23,9 |
| 85 | 92,9 | 42,5 | 1072 |
| 86 | 98,4 | 94,3 | 1,83 |
| 87 | 95,6 | 76,0 | 18,1 |
| 88 | 95,4 | 82,0 | 16,2 |
| 89 | 96,6 | 83,5 | 11,5 |
| 90 | 96,9 | 88,6 | 5,46 |
| 91 | 97,3 | 90,8 | 3,31 |
| 92 | 98,8 | 95,2 | 1,62 |
| 93 | 97,9 | 90,2 | 7,8 |
| 94 | 98,4 | | 2,82 |
| 95 | 98,5 | | 1,45 |
| 96 | 92,3 | | 43,95 |
| 97 | 89,7 | | 98,48 |
| Déviation standard <5% | | | |

| | | | |
|---|---|---|---|
| ¹ Affinité : Exprime le pourcentage de [³H]SQ-29.548 spécifiquement déplacé par le composé examiné. | | | |
| ² IC 50 : Exprime les concentrations requises pour déplacer 50% de [³H]SQ-29.548 lié aux récepteurs TPα. | | | |

Test selon :
Cozzi P., Giordani A., Menichincheri M., Pillan A., Pinciroli V., Rossi A., Tonani R.. Volpi D., Tamburin M.. Ferrario R., Fusar D., Salvati P., - Agents combining thromboxane receptor antagonism with thromboxane synthase inhibition : [[[2-(1H-imidazol-1-yl)ethylidene]amino]oxy]alkanoic acids. - *J. Med. Chem*., **1994**, 37, 3588-3604.

**TABLEAU 3 :**

| Agrégation plaquettaire Résultats du test d'agrégation plaquettaire sur plaquettes humaines | | |
|---|---|---|
| Composé | Test d'agrégation plaquettaire | |
| | Acide arachidonique IC 50¹(µM) | U-46.619 IC 50¹ (µM) |
| Sulotroban | 11,7 | 10.5 |
| SQ-29.548 | 0,035 | 0,034 |
| 18 | 0,36 | 0,48 |
| Déviation standard <5% | | |

| | | |
|---|---|---|
| ¹IC 50 : Exprime les concentrations requises pour réduire de 50% l'agrégation plaquettaire induite par 0,6 nM d'acide arachidonique (AA) ou par 30 nM de U-46619. | | |

Testdécrit selon :
Born G.V.R., Cross M. J., - The agregation of blood platelets. - *J. Physiol*., **1963**. 168, 178-195,
Tsuyoshi T., Masayuki Y., Shuichi W., Kazuhiro K., Takashi Y., -Designe, synthesis, and pharmacology of 3-substituted sodium azulene-1 sulfonates and related compounds : Non-prostaboid thromboxane A₂ receptor antagonists. - *J. Med. Chem*., **1993**, 36, 791-800.

**TABLEAU 4 :**

| Contraction de l'aorte de rat Résultats du test de contraction d'aorte de rat | |
|---|---|
| Composé | Test de contraction d'aorte de rat IC 50 ¹(ηM) |
| Sulotroban | 1.6.10³ |
| SQ-29.548 | 31,8 |
| 17 | 1,38 |
| 18 | 1,21 |
| 22 | 37.6 |
| 25 | 19.7 |
| 29 | 20.6 |
| 40 | 17,7 |
| Déviation standard <5% | |

| | |
|---|---|
| ¹IC 50 : Exprime les concentrations en composé réduisant de 50% l'intensité du tonus musculaire de l'aorte de rat induit par le U-46619 (0,03 µM). | |

Test décrit selon :
de Tullio P., Pirotte B., Lebrun P., Fontaine J., Dupont L.. Antoine M. H., Ouedraogo R.. Khelili S., Maggetto C., Masereel B., Diouf O., Podona T., Delarge J., 3-and-4-substituted 4H-pyrido[4,3-e]-1,2,4-thiadiazine 1,1-dioxides as potassium channels openers : synthesis pharmacological evaluation, and structure-activity relationships. - J. *Med. Chem.,* **1996**, 39, 937-948.

**TABLEAU 5 :**

| Contraction de fundus de rat Résultats du test de prévention de contraction de fundus de rat | |
|---|---|
| Composé | Test de prévention de contraction de fundus de rat IC 50 ¹ (µM) |
| Sulotroban | 0,83 |
| SQ-29.548 | 0,18 |
| 18 | 0,07 |
| Déviation standard <5% | |

| | |
|---|---|
| ¹IC 50 : Exprime les concentrations en composé réduisant de 50% l'amplitude maximale de la contraction induite par 5 µg de U-46619. | |

Test décrit selon :
Harris N., Greenberg R., Phillips M. B., Michel I. M., Goldenberg H. J., Haslanger M. F., Steinbacher T.E., - Effects of SQ-27,427, a thromboxane A2 receptor antagonist, in the human platelet and isolated smooth muscle. - *Eur. J. Pharmacol*., **1984**, 103, 9-18.

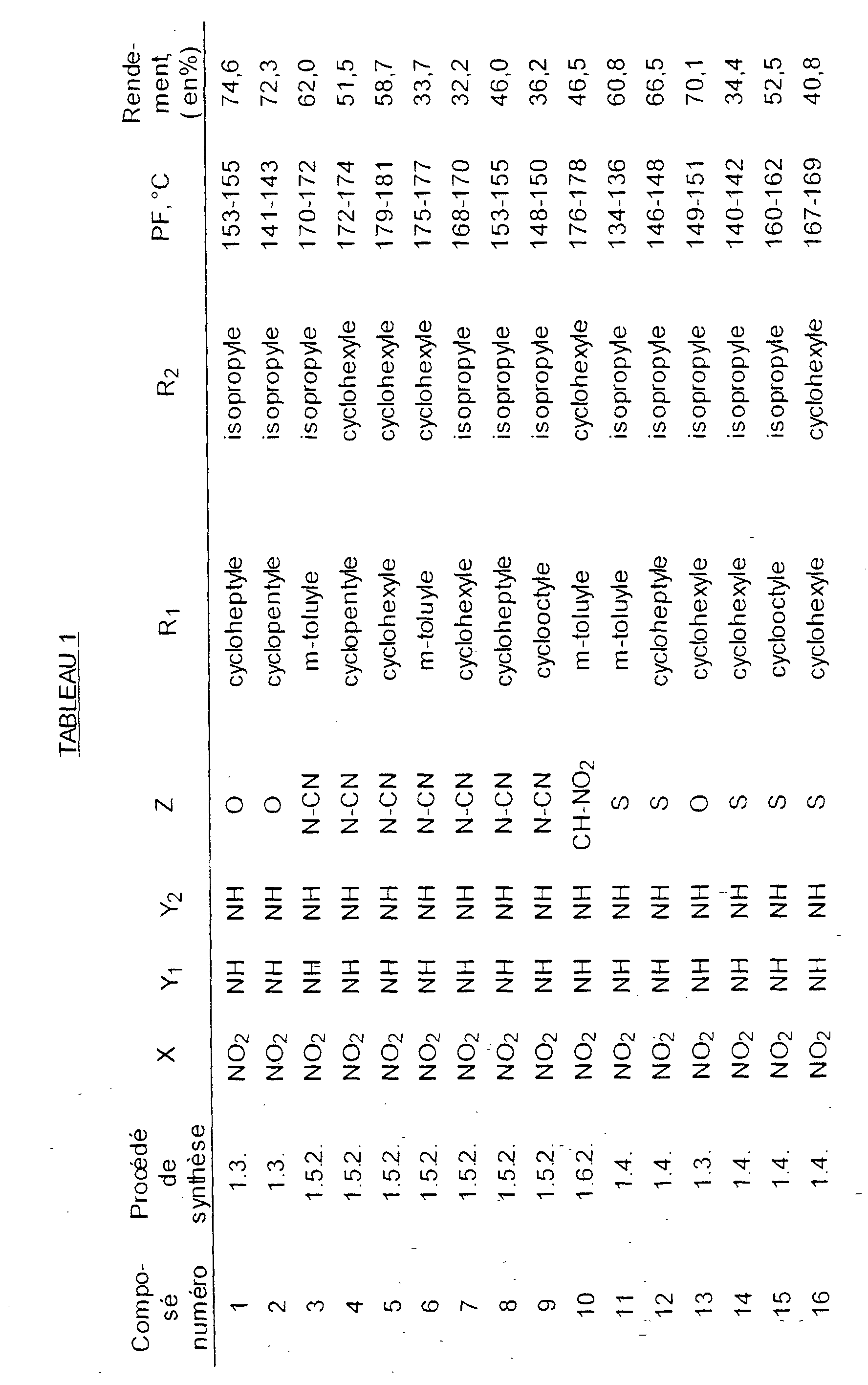

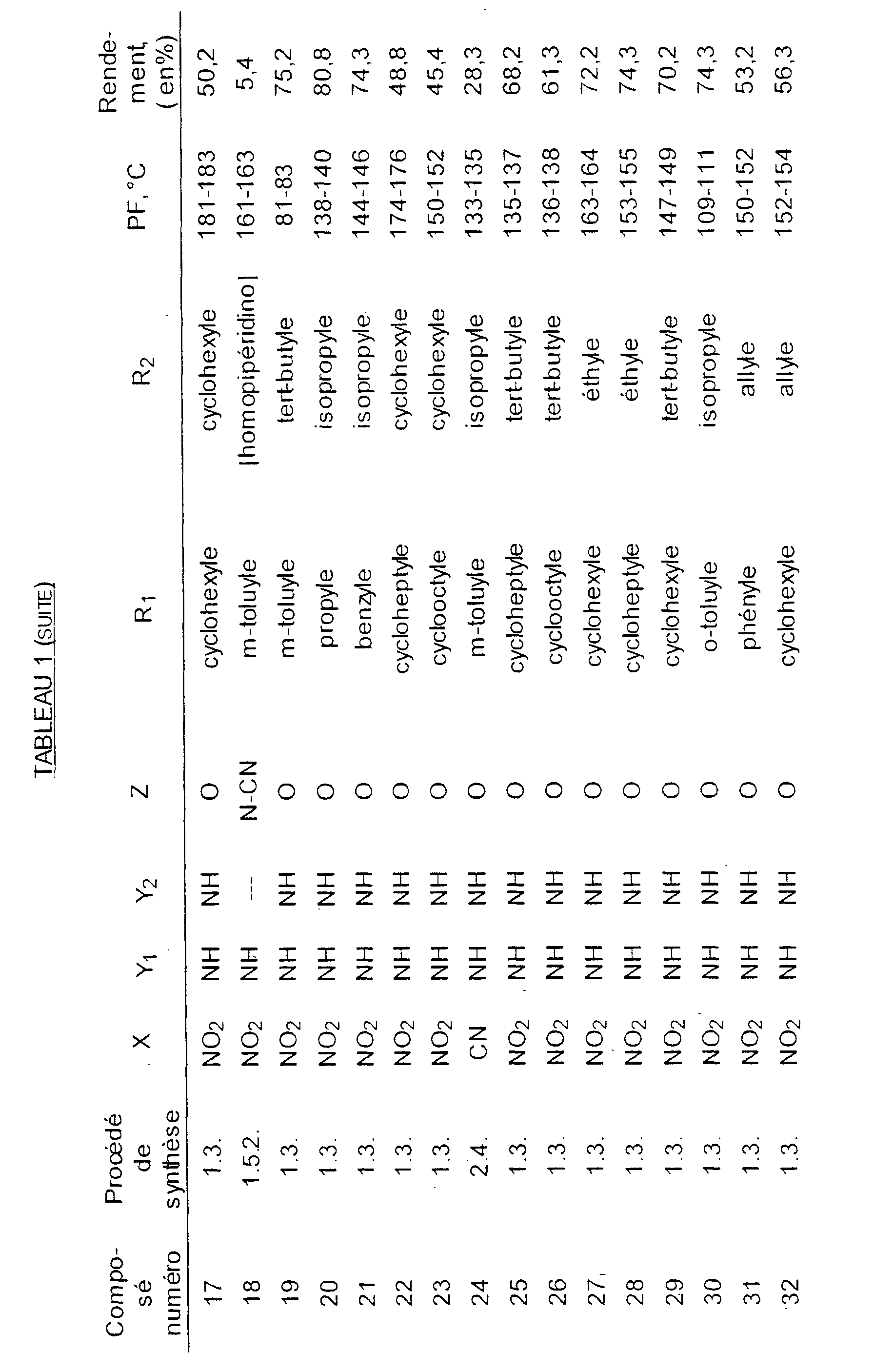

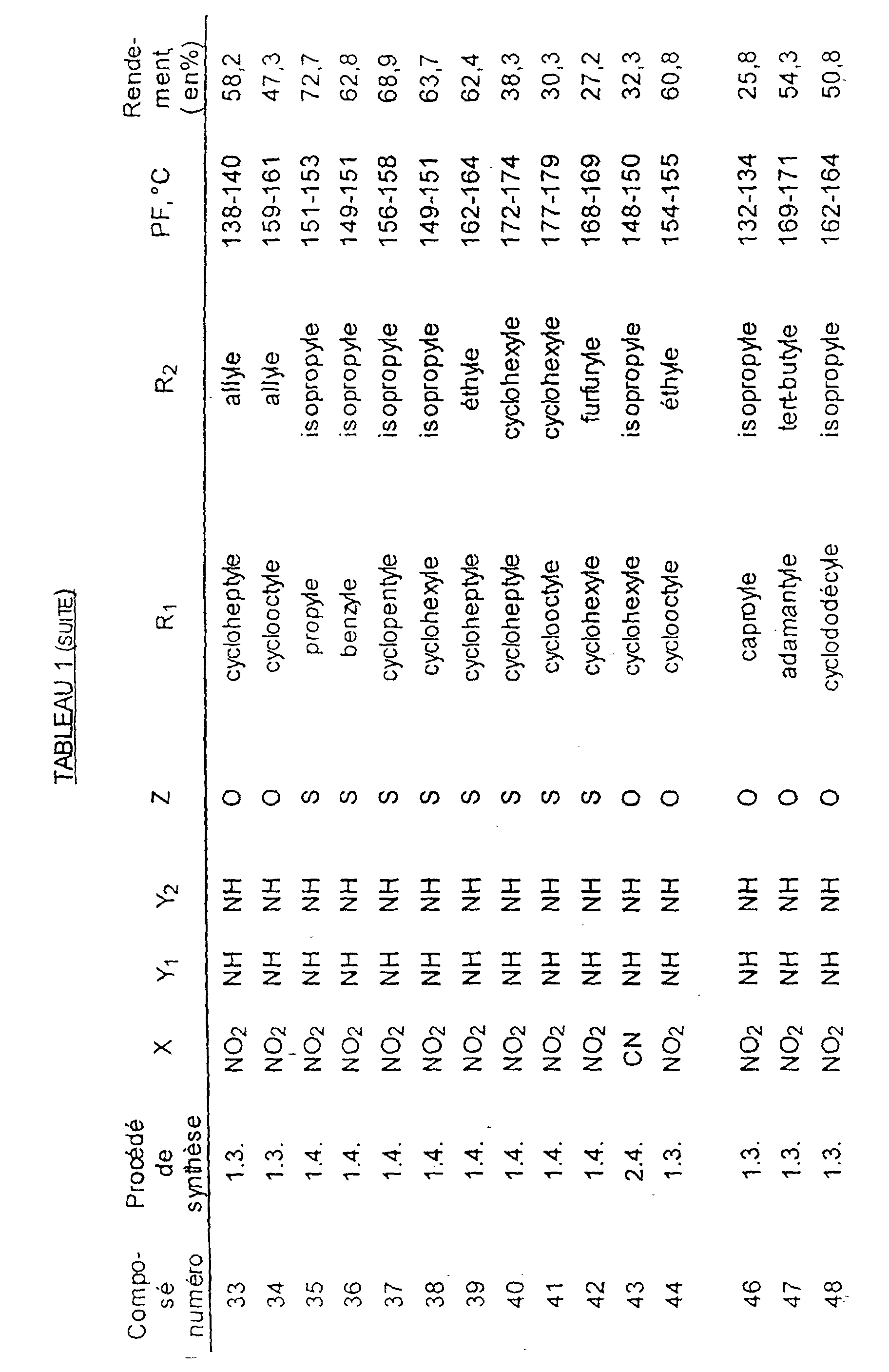

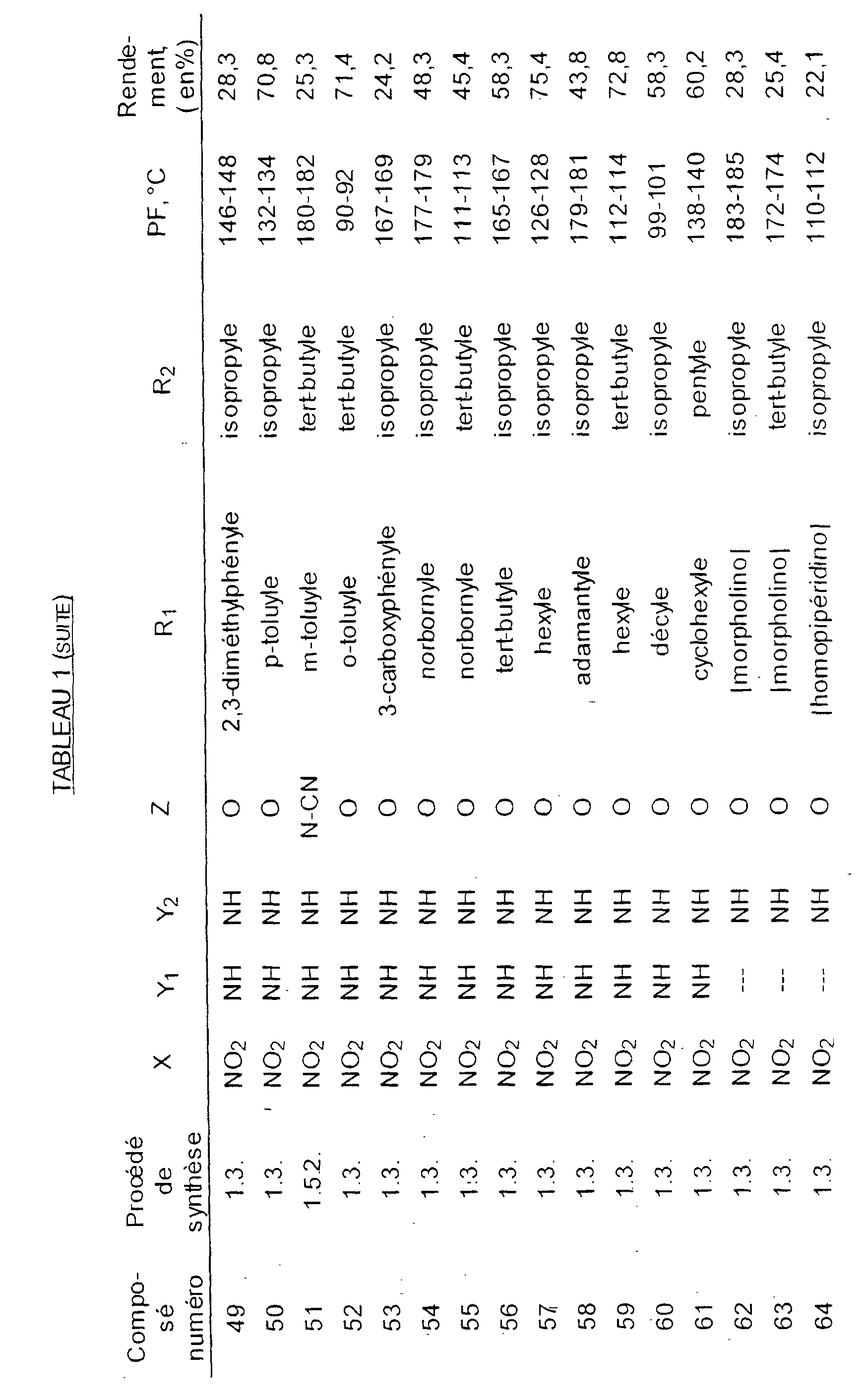

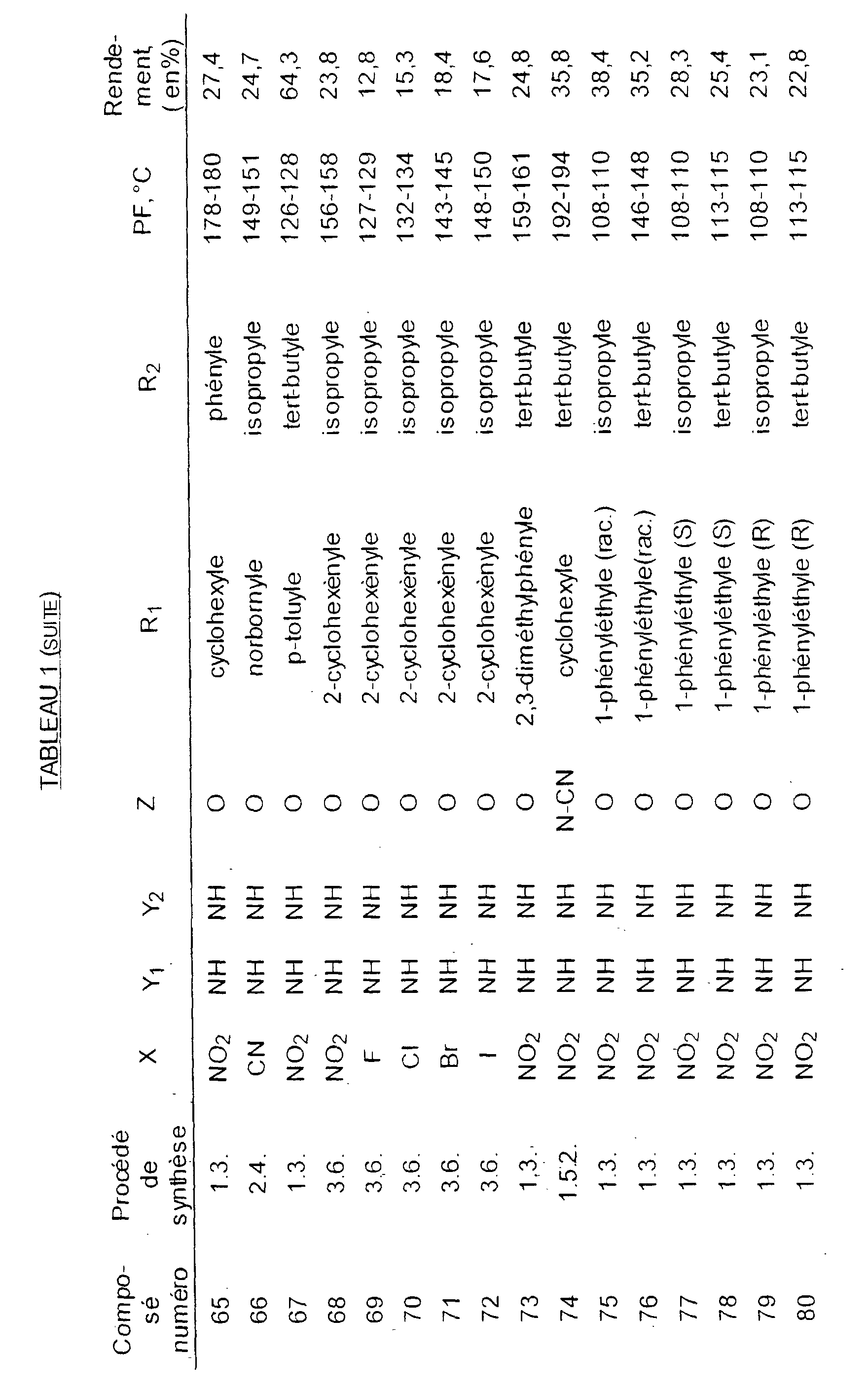

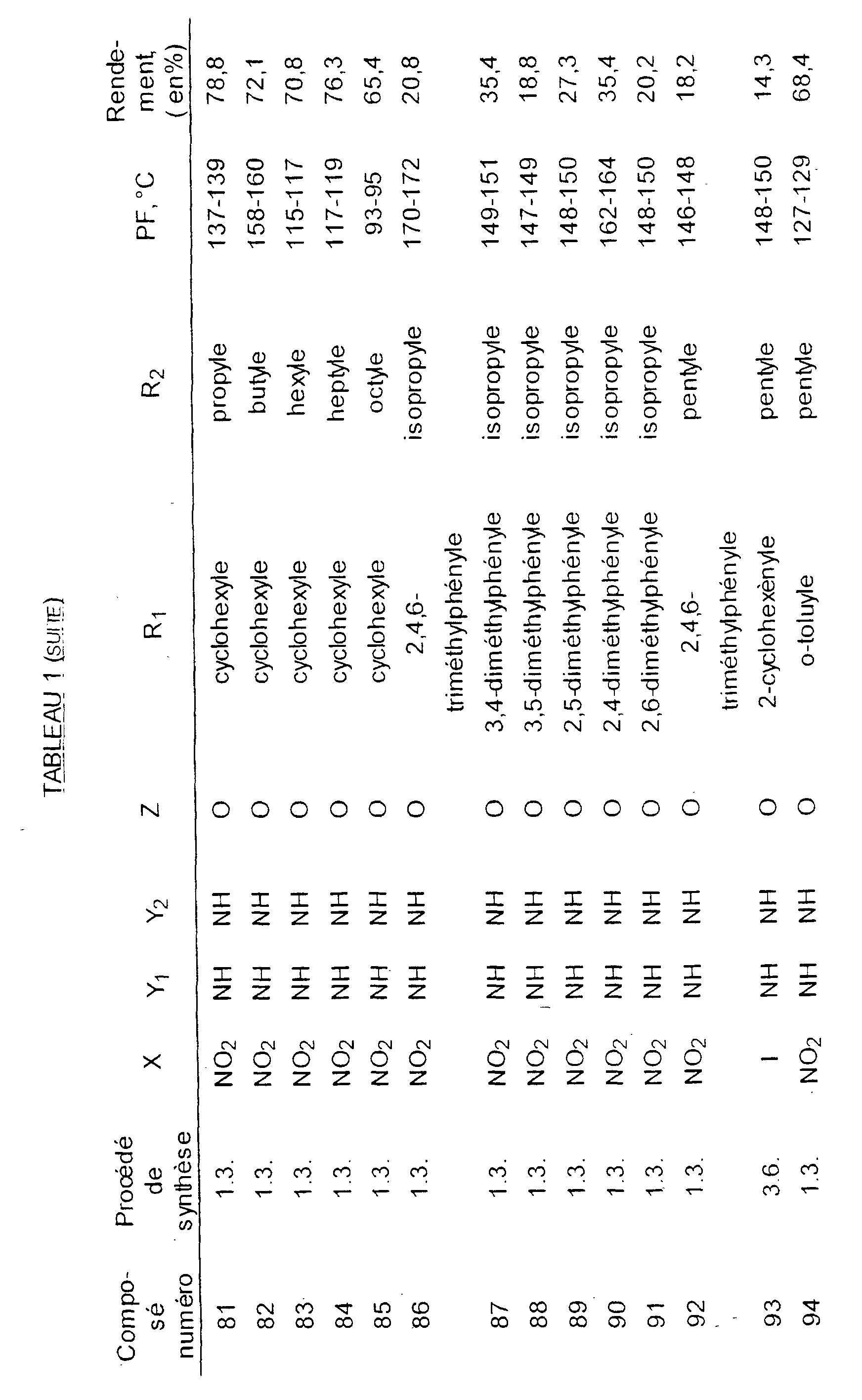

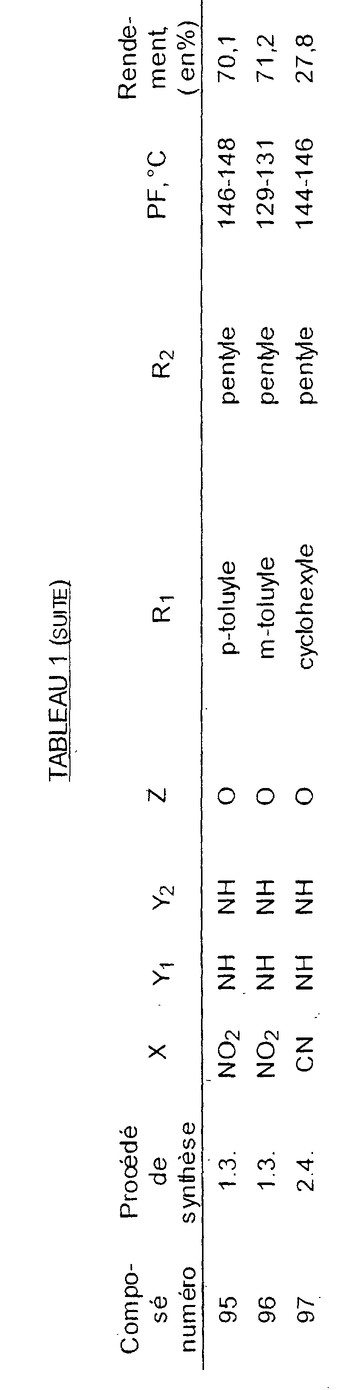

## Revendications

1. Dérivé de sulfonamides benzéniques répondant à la formule générale (I) : 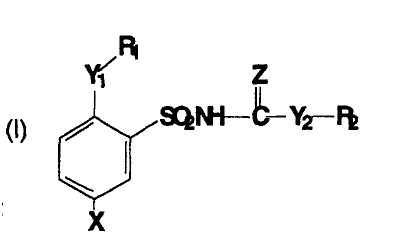 dans laquelle
X représente un groupe nitro, cyano, halogéno, éventuellement radioactif.
Y₁ représente un groupe aminé secondaire ou tertiaire, du soufre ou de l'oxygène;
Y₂ représente un groupe -NH, ou de l'azote ;
Z représente de l'oxygène, du soufre, -N-CN ou -CH-NO₂; et
R₁ et R₂, qui peuvent être identiques ou différents, représentent chacun indépendamment un groupe alkyle linéaire ou ramifié, saturé ou insaturé, de 2 à 12 atomes de carbone, un groupe alicyclique, saturé ou insaturé, de 3 à 12 atomes de carbone, éventuellement radioactif, un groupe aryle, substitué ou non par un ou plusieurs groupes alkyle en C₁-C₄, nitro, cyano, trifluorométhyle, carboxy et halogène, ou un groupe arylalkyle,
ou bien R₁ et/ou R₂ forment avec Y₁ et/ou Y₂ un groupe hétérocyclique de 5 à 7 chaînons, saturé ou insaturé
à l'exception des dérivés pour lesquels X est un groupe nitro, Y₁ représente un groupe amine secondaire (-NH-), Y₂ représente un groupe -NH , Z un oxygène, R₂, un isopropyle et R₁ un élément sélectionné dans le groupe constitué de (m-toluyle, phenyle et cyclooctyle) et à l'exception du N-[(2-CYCLOOCTYLAMINO-5-CYANOBENZENE)SULFONYL] N'-ISOPROPYL UREE. ;

2. Dérivé suivant la revendication 1, **caractérisé en ce que** X représente groupe nitro, cyano, bromo, iodo.

3. Dérivé suivant l'une ou l'autre des revendications 1 et 2, **caractérisé en ce que** Y₁ représente un groupe -NH et Y₂ représente un groupe -NH ou un atome d'oxygène.

4. Dérivé suivant l'une quelconque des revendications 1 à 3, **caractérisé en ce que** R₁ et R₂ représentent chacun indépendamment un groupe éthyle, butyle, tert-butyle, propyle, isopropyle, pentyle, hexyle, heptyle, octyle, décyle, amyle, cyclopropyle, cyclobutyle, cyclopentyle, cyclohexyle, cycloheptyle, cyclooctyle, cyclododécyle, 2-cyclohexènyle, m-toluyle, o-toluyle, p-toluyle, phényle, allyle, adamantyle, norbornyle; caproyle, 3-carboxyphényle, 2,3-diméthylphényle, 2,4-diméthylphényle, 2,5-diméthylphényle, 2,6-diméthylphényle, 3,4-diméthylphényle, 3,5-diméthylphényle, 2,4,6-triméthylphényle, furfuryle, benzyle ou 1-phényléthyle.

5. Dérivé suivant l'une ou l'autre des revendications 1 et 2, **caractérisé en ce que** R₂ et Y₂ forment un groupe homopipéridino.

6. Dérivé suivant l'une ou l'autre des revendications 1 et 2, **caractérisé en ce que** R₁ et Y₁ forment un groupe morpholino ou homopipéridino.

7. Dérivé suivant l'une quelconque des revendications 1 à 6, **caractérisé en ce qu'**il est constitué par un sel choisi dans le groupe formé par les sels sodiques, les sels potassiques et les sels d'acides aminés tels que lysine, arginine.

8. Dérivé suivant l'une quelconque des revendications 1 à 7, **caractérisé en ce qu'**il est choisi dans le groupe comprenant :
la N-[(2-cyclohexylamino-5-nitrobenzène)sulfonyl]N'-tert-butyl urée,
la N-cyano-N'-[(2-métatoluylamino-5-nitrobenzéne)sulfonyl]homopipéridinoamidine,
la N-[(2-cycloheptylamino-5-nitrobenzène)sulfonyl]N'-cyclohexyl thiourée, et
la N-[(cyclohexèn-2-yl)-5-iodobenzène)sulfonyl]N'-pentyl urée.

9. Composition pharmaceutique, **caractérisée en ce qu'**elle comprend un dérivé de sulfonamide benzénique suivant l'une quelconque des revendications 1 à 8 en mélange avec un excipient pharmaceutique acceptable et éventuellement d'autres agents thérapeutiques.

10. Utilisation d'un dérivé suivant l'une quelconque des revendications 1 à 8, pour la fabrication d'un médicament pour le traitement et/ou la prévention des maladies impliquant le thromboxane A₂,

11. Utilisation selon la revendication 10 pour le traitement et/ou la prévention des maladies impliquant le thromboxane A2 au niveaux cardio-vasculaire et sanguins.

12. Utilisation selon la revendication 10 pour le traitement et/ou la prévention des maladies impliquant le thromboxane A2 au niveaux pulmonaires, au niveaux de la reproduction ou au niveaux rénaux.

13. Utilisation d'un dérivé radiomarqué suivant l'une quelconque des revendications 1 à 8, comme liaison au récepteur du thromboxane A₂ dans un test pharmacologique hors du corps.

## Claims

1. Benzene-sulphonamide derivatives having the general formula (I): 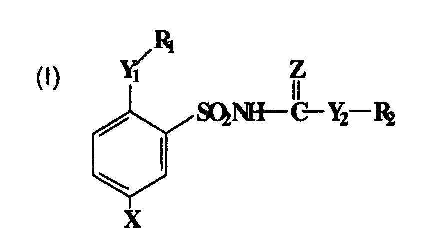 in which:
X represents a nitro, cyano, or optionally radio-labeled halogen group,
Y₁ represents a secondary or tertiary amino group, a sulphur or an oxygen
Y₂ represents a -NH group or a nitrogen;
Z represents oxygen, sulphur, -N-CN or -CH-NO₂; and
R₁ and R₂, which can be identical or different, represent each independently a saturated or unsaturated optially radio-labeled linear or branched alkyl group with 2 to 12 carbon atoms, a saturated or unsaturated optinally radiolabeled alicyclic. group with 3 to 12 carbon atoms, an aryl group substituted or not by one or several alkyl groups in C₁-C₄, nitro, cyano, trifluoromethyl, carboxy and halogen groups, or an arylalkyl group,
or R₁ and/or R₂ form with Y₁ and/or Y₂ a saturated or unsaturated heterocyclic group having 5 to 7 ring members,
with the exception of derivatives for which X is a nitro group, Y₁ represents a secondary amine group (-NH-), Y₂ represents a -NH group, Z an oxygen, R₂, an isopropyl and R₁ an element selected from a group consisting of (m-toluyl, phenyl and cyclooctyl) and with the exception of N-[(2-CYCLOOCTYLAMiNO-5-CYANOBeNZeNe)SULFONYL] N'-isopropyl urea. ;

2. Derivative according to claim 1, **characterized in that** X represents nitro, cyano, bromo, iodine group.

3. Derivative according to one or the other claims 1 and 2, **characterized in that** Y₁ represents a -NH group and Y₂ represents a -NH group or an oxygen atom.

4. Derivative according to any of claims 1 to 3, **characterized in that** R₁ and R₂ represent each independently an ethyl, butyl, tert-butyl, propyl, isopropyl, pentyl, hexyl, heptyl, octyl, decyl, amyl, cyclopropyl, cyclobutyl, cyclopentyl, cyclohexyl, cycloheptyl, cyclooctyl, cyclododecyl, 2-cyclohexenyl, m-toluyl, o-toluyl, p-toluyl, phenyl, allyl, adamantyl, norbomyl; caproyl, 3-carboxyphenyl, 2,3-dimethylphenyl, 2,4-dimethylphenyl, 2,5-dimethylphenyl, 2,6-dimethylphenyl, 3,4-dimethylphenyl, 3,5-dimethylphenyl, 2,4,6-trimethylphenyl, furfuryl, benzyl or 1-phenylthyl group.

5. Derivative according to one or the other claims 1 and 2, **characterized in that** R₂ and Y₂ form a homopiperidin group.

6. Derivative according to one or the other claims 1 and 2, **characterized in that** R₁ and Y₁ form a morpholin or homopiperidin group.

7. Derivative according to any of claims 1 to 6, **characterized in that** it is constituted by a salt chosen into the group formed by sodium salts, the potassic salts and the amino acid salts such as lysine, arginine.

8. Derivative according to any of claims 1 to 7, **characterized in that** it is chosen in a group comprising:
N-[(2-cyclohexylamino-5-nitrobenzene)sulfonyl]N'-tert-butyl urea,
N-cyano-N'-[(2-metatoluylamino-5-nitrobenzene)sulfonyl]homopiperidinoamidine,
N-[(2-cycloheptylamino-5-nitrobenzene)sulfonyl]N'-cyclohexyl thiourea, and
N-[(cyclohexen-2-yl)-5-iodobenzene)sulfonyl]N'-pentyl urea.

9. Pharmaceutical composition, **characterized in that** it comprises a benzene sulphonamide derivative according to any of claims 1 to 8 in mixture with an acceptable pharmaceutical excipient and optionally other therapeutic agents.

10. Use of a derivate according to any of claims 1 to 8, for producing a medication for treatment and/or prevention of an illness involving a thromboxan A2 ,

11. Use according to claim 10 for treatment and/or prevention of an illness involving a thromboxan A2 at cardio-vascular and blood levels,

12. Use according to claim 10 for treatment and/or prevention of an illness involving a thromboxan A2 at pulmonary, reproduction or renal levels.

13. Use of a radio-labeled derivative according to any of claims 1 to 8, as binding to thromboxan A2 receptor in a pharmacological outside body test..

## Patentansprüche

1. Benzolsulfonamidderivate der allgemeinen Formel (I): 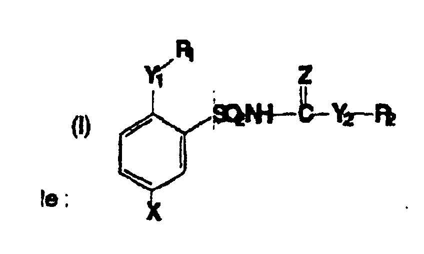 worin:
X für eine Nitro-, Cyano- oder optional radioaktive Halogengruppe steht;
Y₁ für eine sekundäre oder tertiäre Aminogruppe, Schwefel oder Sauerstoff steht;
Y₂ für eine -NH-Gruppe oder Stickstoff steht;
Z für Sauerstoff, Schwefel, -N-CN oder -CH-NO₂ steht
und
R₁ und R₂ identisch oder verschieden sein können und jeweils unabhängig voneinander für eine gesättigte oder ungesättigte optional radioaktive lineare oder verzweigte Alkylgruppe mit 2 bis 12 Kohlenstoffatomen, eine gesättigte oder ungesättigte optional radioaktive alicyclische Gruppe mit 3 bis 12 Kohlenstoffatomen, durch eine oder mehrere C₁-C₄-Alkyl-, Nitro-, Cyano-, Trifluormethyl-, Carboxyl- und Halogengruppen substituierte oder nicht Arylgruppe oder eine Arylalkylgruppe stehen,
oder R₁ und/oder R₂ mit Y₁ und/oder Y₂ eine gesättigte oder ungesättigte 5- bis 7-gliedrige heterocyclische Gruppe bilden,
mit Ausnahme derjenigen Derivate, bei denen X für eine Nitrogruppe steht, Y₁ für eine sekundäre Aminogruppe (-NH-) steht, Y₂ für eine -NH-Gruppe steht, Z für Sauerstoff steht, R₂ für Isopropyl steht und R₁ für ein Element aus der Gruppe bestehend aus *m*-Toluyl, Phenyl und Cyclooctyl steht, und mit Ausnahme von N-[(2-Cyclooctylamino-5-cyanobenzol)sulfonyl]-N'-isopropylhamstoff.

2. Derivate nach Anspruch 1, **dadurch gekennzeichnet, daß** X für eine Nitro-, Cyano-, Brom- oder lodgruppe steht.

3. Derivate nach Anspruch 1 oder 2, **dadurch gekennzeichnet, daß** Y₁ für eine -NH-Gruppe steht und Y₂ für eine -NH-Gruppe oder ein Sauerstoffatom steht.

4. Derivate nach einem der Ansprüche 1 bis 3, **dadurch gekennzeichnet, daß** R₁ und R₂ jeweils unabhängig voneinander für eine Ethyl-, Butyl-, tert.-Butyl-, Propyl-, Isopropyl-, Pentyl-, Hexyl-, Heptyl-, Octyl-, Decyl-, Amyl-, Cylopropyl-, Cyclobutyl-, Cyclopentyl-, Cyclohexyl-, Cycloheptyl-, Cyclooctyl-, Cyclododecyl-, 2-Cyclohexenyl-, m-Toluyl-, o-Toluyl-, p-Toluyl-, Phenyl-, Allyl-, Adamantyl-, Norbomyl-, Caproyl-, 3-Carboxyphenyl-, 2,3-Dimethylphenyl-, 2,4-Dimethylphenyl-, 2,5-Dimethylphenyl-, 2,6-Dimethylphenyl-, 3,4-Dimethylphenyl-, 3,5-Dimethylphenyl-, 2,4,6-Trimethylphenyl-, Furfural-, Benzyl- oder 1-Phenylethylgruppe stehen.

5. Derivate nach Anspruch 1 oder 2, **dadurch gekennzeichnet, daß** R₂ und Y₂ eine Homopiperidinogruppe bilden.

6. Derivate nach Anspruch 1 oder 2, **dadurch gekennzeichnet, daß** R₁ und Y₁ eine Morpholino- oder Homopiperidinogruppe bilden.

7. Derivate nach einem der Ansprüche 1 bis 6, **dadurch gekennzeichnet, daß** es sich um ein Salz aus der Gruppe bestehend aus Natriumsalzen, Kaliumsalzen und Salzen von Aminosäuren, wie Lysin und Arginin, handelt.

8. Derivate nach einem der Ansprüche 1 bis 7, **dadurch gekennzeichnet, daß** es aus der Gruppe, bestehend aus:
N-[(2-Cyclohexylamino-5-nitrobenzol)sulfonyl]-N'-tert.butylhamstoff,
N-Cyano-N'-[(2-meta-toluylamino-5-nitrobenzol)-sulfonyl]homopiperidinoamidin
N-[(2-Cycloheptylamino-5-nitrobenzol)sulfonyl]-N'cyclohexylthiohamstoff und
N-[(Cyclohexen-2-yl)-5-iodbenzol)sulfonyl]-N'-pentylhamstoff,
ausgewählt ist.

9. Pharmazeutische Zusammensetzung, **dadurch gekennzeichnet, daß** sie ein Benzolsulfonamidderivat nach einem der Ansprüche 1 bis 8 in Abmischung mit einem pharmazeutisch annehmbaren Träger und optional anderen therapeutischen Hilfsmitteln enthält.

10. Verwendung eines Derivats nach einem der Ansprüche 1 bis 8 zur Herstellung eines Arzneimittels zur Behandlung und/oder Prävention von Erkrankungen, bei denen Thromboxan A₂ eine Rolle spielt.

11. Verwendung nach Anspruch 10 zur Behandlung und/oder Prävention von Herz-Kreislauf- und Bluterkrankungen, bei denen Thromboxan A₂ eine Rolle spielt.

12. Verwendung nach Anspruch 10 zur Behandlung und/oder Prävention von Lungen-, Reproduktions- oder Nierenerkrankungen, bei denen Thromboxan A₂ eine Rolle spielt.

13. Verwendung eines radioaktiv markierten Derivats nach einem der Ansprüche 1 bis 8 zur Bindung an den Thromboxan-A₂-Rezeptor in einem exkorporalen pharmakologischen Test.
